# EUROPÄISCHE PATENTANMELDUNG

(11) **EP 1 813 291 A1**
(43) Veröffentlichungstag der Anmeldung: **01.08.2007**
(21) Anmeldenummer: 06100673.0
(22) Anmeldetag: 20.01.2006
(51) Int. Cl.: A61L 15/46, A61L 15/60

(54) **Geruchsverhindernde wasserabsorbierende Zusammensetzungenen enthaltend Urease-Inhibitoren**

(71) Anmelder: BASF Aktiengesellschaft, 67056 Ludwigshafen (DE)
(72) Erfinder: Braig, Volker, 69469, Weinheim-Lützelsachsen (DE); Marco, Michael, 69469, Weinheim (DE)

(57) **Zusammenfassung**

Die Erfindung betrifft geruchsverhindernde wasserabsorbierende Zusammensetzungen, enthaltend mindestens ein wasserabsorbierendes Polymer und mindestens einen Urease-Inhibitor, wobei das Polymer ein Säure-Gruppen tragendes Polymer ist, dessen Säure-Gruppen zu 50 bis 65 mol-% neutralisiert sind, und der Gehalt an Urease-Inhibitor von 0,0001 bis 0,1 Gew.-%, bezogen auf die Zusammensetzung, beträgt, Verfahren zur Herstellung der Zusammensetzung sowie Hygieneartikel und deren Herstellung.

## Beschreibung

Die vorliegende Erfindung betrifft geruchsverhindernde wasserabsorbierende Zusammensetzungen, enthaltend mindestens ein wasserabsorbierendes Polymer und mindestens einen Urease-Inhibitor, Verfahren zu deren Herstellung sowie Hygieneartikel und deren Herstellung.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemä-βen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden, Inkontinenzprodukten und anderen Hygioneartikeln, aber auch als wasserzurückhaltende Mittel im landwirtschaftlichen Gartenbau verwendet.

In Hygieneartikeln können während der Anwendung unangenehme Gerüche entstehen, beispielsweise durch Zersetzung von Harnstoff. In WO-A-98/26808, WO-A-03/053486, JP-A-2001/258934, EP-A-0 739 635 und EP-A-1 034 800 und werden verschiedene Lösungen des Problems vorgeschlagen.

WO-A-98/26808 beschreibt absorbierende Zusammensetzungen, die ein beliebiges Absorptionsmittel für Flüssigkeiten, ein Absorptionsmittel für Gerüche sowie eine oder mehrere Substanzen aus der Gruppe Biozide, Urease-Inhibitoren und pH-Regulatoren enthalten. Als bevorzugte Absorptionsmittel für Flüssigkeiten werden vernetzte Polyacrylsäuren mit einem Neutralisationsgrad von mindestens 75 mol-% genannt.

WO-A-03/053486 offenbart die Verwendung von Yucca-Extrakt als Urease-Inhibitor.

JP-A-2001/258934 lehrt die Verwendung schwach saurer wasserabsorbierender Polymere mit einem Neutralsationsgrad von 40 bis 65 mol-% in Kombination mit Urease-Inhibitoren. JP-A-2001/258934 enthält keinen Hinweis auf den überproportionalen Abfall der Absorptionskapazität bei Verwendung von mehr als 0,1 Gew.-% des Urease-Inhibitors.

EP-A-0 739 635 beschreibt absorbierende Zusammensetzungen, die Borsäuresalze enthalten.

EP-A-1 034 800 beschreibt die Verwendung von Kombinationen aus Geruchsbinder und Oxidationsmittel zur Vermeidung von unangenehmen Gerüchen

Aufgabe der vorliegenden Erfindung war die Bereitstellung verbesserter wasserabsorbierender Zusammensetzungen, die nach Beladung mit Urin oder anderer Körperflüssigkeiten unangenehme Gerüche zuverlässig verhindern.

Eine weitere Aufgabe der vorliegenden Erfindung war die Bereitstellung geruchsverhindernder wasserabsorbierender Zusammensetzungen, die lagerstabil sind, d.h. die Zusammensetzungen sollen sich bei längerer Lagerung weder verfärben noch ihre geruchsverhindernde Wirkung verlieren.

Gelöst wurde die Aufgabe durch Zusammensetzungen, enthaltend mindestens ein wasserabsorbierendes Polymer und mindestens einen Urease-Inhibitor, wobei das Polymer ein Säure-Gruppen tragendes Polymer ist, dessen Säure-Gruppen zu 50 bis 65 mol-% neutralisiert sind, und der Gehalt an Urease-Inhibitor von 0,0001 bis 0,1 Gew.-%, bezogen auf die Zusammensetzung, beträgt.

Die Säure-Gruppen des wasserabsorbierenden Polymeren sind vorzugsweise zu 52 bis 63 mol-%, besonders bevorzugt zu 54 bis 61 mol-%, ganz besonders bevorzugt zu 55 bis 60 mol-%, neutralisiert.

Die erfindungsgemäße Zusammensetzung enthält üblicherweise mindestens 90 Gew.-%, vorzugsweise mindestens 95 Gew.-%, besonders bevorzugt mindestens 99 Gew.-%, des mindestens einen wasserabsorbierenden Polymeren.

Das mindestens eine wasserabsorbierende Polymer ist vorzugsweise ein Polymer auf Basis einer vernetzten Acrylsäure.

Das mindestens eine wasserabsorbierende Polymer liegt vorzugsweise in Form oberflächennachvernetzter Partikel vor.

Die wasserabsorbierenden Polymere werden beispielsweise durch Polymerisation einer Monomerlösung, enthaltend
a) mindestens ein ethylenisch ungesättigtes, säuregruppentragendes Monomer,
b) mindestens einen Vernetzer,
c) gegebenenfalls ein oder mehrere mit dem Monomeren a) copolymerisierbare ethylenisch und/oder allylisch ungesättigte Monomere und
d) gegebenenfalls ein oder mehrere wasserlösliche Polymere, auf die die Monomere a), b) und ggf. c) zumindest teilweise aufgepfropft werden können,
erhalten.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylother (M EHQ) und/oder Tocopherole.

Unter Tocopherol werden Verbindungen der folgenden Formel verstanden 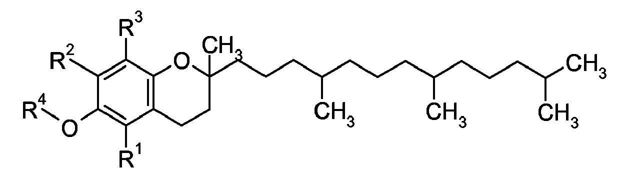 wobei R¹ Wasserstoff oder Methyl, R² Wasserstoff oder Methyl, R³ Wasserstoff oder Methyl und R⁴ Wasserstoff oder einen Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A-0 530 438 beschrieben, Di- und Triacrylate, wie in EP-A-0 547 847, EP-A-0 559 476, EP-A-0 632 068, WO-A-93/21237, WO-A-03/104299, WO-A-03/104300, WO-A-03/104301 und DE-A-103 31 450 beschrieben, gemischte Acrylate, die neben Acrylatgruppen weitere ethylenisch ungesättigte Gruppen enthalten, wie in DE-A-103 31 456 und WO-A-04/013064 beschrieben, oder Vernetzermischungen, wie beispielsweise in DE-A-195 43 368, DE-A-196 46 484, WO-A-90/15830 und WO-A-02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO-A-03/104301 beschrieben sind. Besonders vorteilhaft sind Di- und/oder Triacrylate des 3- bis 10-fach ethoxylierten Glyzerins.

Ganz besonders bevorzugt sind Di- oder Triacrylate des 1- bis 5- fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Menge an Vernetzer b) beträgt vorzugsweise mindestens 0,001 mol-%, besonders bevorzugt mindestens 0,005 mol-%, ganz besonders bevorzugt mindestens 0,01 mol-%, und vorzugsweise bis zu 10 mol-%, besonders bevorzugt bis zu 5 mol-%, ganz besonders bevorzugt bis zu 2 mol-%, jeweils bezogen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Daher können die Polymerisationsinhibitoren vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten wasserabsorbierenden Polymers sowie weitere geeignete hydrophile ethylenisch ungesättigte Monomere d) werden in DE-A-199 41 423, EP-A-0 686 650, WO-A-01/45758 und WO-A-03/104300 beschrieben.

Wasserabsorbierende Polymere werden üblicherweise durch Polymerisation einer wässrigen Monomerlösung und gegebenenfalls einer anschließenden Zerkleinerung des Hydrogels erhalten. Geeignete Herstellverfahren sind in der Literatur beschrieben. Wasserabsorbierende Polymere können beispielsweise erhalten werden durch
- Gelpolymerisation im Batchverfahren bzw. Rohrreaktor und anschließender Zerkleinerung im Fleischwolf, Extruder oder Kneter (EP-A-0 445 619, DE-A-19 846 413)
- Polymerisation im Kneter, wobei durch beispielsweise gegenläufige Rührwellen kontinuierlich zerkleinert wird, (WO-A-01/38402)
- Polymerisation auf dem Band und anschließende Zerkleinerung im Fleischwolf, Extruder oder Kneter (DE-A-38 25 366, US-6,241,928)
- Emulsionspolymerisation, wobei bereits Perlpolymerisate relativ enger Golgrößenverteilung anfallen (EP-A-0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO-A-02/94328, WO-A-02/94329)
- Sprühpolymerisation (WO-A-96/40427, DE-A-103 40 253)

Die Umsetzung wird vorzugsweise in einem Kneter, wie beispielsweise in WO-A-01/38402 beschrieben, oder auf einem Bandreaktor, wie beispielsweise in EP-A-0 955 086 beschrieben, durchgeführt.

Die Säuregruppen der erhaltenen Hydrogele sind zu 50 bis 65 mol-%, vorzugsweise zu 52 bis 63 mol-%, bevorzugt zu 54 bis 61 mol-%, besonders bevorzugt zu 55 bis 60 mol-%, neutralisiert, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium als Alkalimetalle besonders bevorzugt sind, ganz besonders bevorzugt jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Neutralisation kann nach der Polymerisation auf der Stufe des Hydrogels durchgeführt werden. Es ist aber auch möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso weißer, wenn der Feststoffgehalt des Gels möglichst hoch ist. Bevorzugt liegt der Feststoffgehalt des Geles vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 850 µm, und vorzugsweise über 80 µm, besonders bevorzugt über 90 µm, ganz besonders bevorzugt über 100 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die wasserabsorbierende Polymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyepoxide, wie in EP-A-0 083 022, EP-A-543 303 und EP-A-937 736 beschrieben, di- oder polyfunktionelle Alkohole, wie in DE-C-33 14 019, DE-C-35 23 617 und EP-A-450 922 beschrieben, oder β-Hydroxyalkylamide, wie in DE-A-102 04 938 und US-6,239,230 beschrieben.

Des weiteren sind in DE-A-40 20 780 zyklische Karbonate, in DE-A-198 07 502 2-Oxazolidon und dessen Derivate, wie 2-Hydroxyethyl-2-oxazolidon, in DE-A-198 07 992 Bis- und Poly-2-oxazolidinone, in DE-A-198 54 573 2-Oxotetrahydro-1,3-oxazin und dessen Derivate, in DE-A-198 54 574 N-Acyl-2-Oxazolidone, in DE-A-102 04 937 zyklische Harnstoffe, in DE-A- 103 34 584 bizyklische Amidacetale, in EP-A-1 199 327 Oxetane und zyklische Harnstoffe und in WO-A-03/031482 Morpholin-2,3-dion und dessen Derivate als geeignete Oberflächennachvernetzer beschrieben.

Vorteilhaft werden polyvalente Kationen neben den Oberflächennachvernetzern zur Oberflächennachvernetzung verwendet. Die einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt.

Die Nachvernetzung wird üblicherweise so durchgeführt, dass eine Lösung des O-berflächennachvernetzers auf das Hydrogel oder das trockene Polymerpulver aufgesprüht wird. Dabei können Oberflächennachvernetzer und polyvatentes Kation in einer gemeinsamen Lösung oder als getrennte Lösungen aufgesprüht werden. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer. Ganz besonders bevorzugt werden Hochgeschwindigkeitsmischer, beispielsweise vom Typ Schuggi-Flexomix® oder Turbolizer® eingesetzt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

Geeignete Urease-Inhibitoren sind 2-Brom-2-nitro-1,3-propandiol (Bronopol), Triclosan, substituierte Thiophosphorsäuretriamide, Borsäure und dessen Derivate, Hydroxaminsäurederivate, Cystamin und dessen Derivate, Dicumerol und dessen Derivate, Hydrochinon und dessen Derivate, Fluoride, N-Alkylharnstoffe, N-Arylharnstoffe, 5-methoxy-2-[[(4-methoxy-3,5-dimethyl-2-pyridinyl)methyl]sulfinyl]-1H-benzimidazol (Omeprazol), Oxalsäuredihydrazid, Polyvinylpyrrolidon-Jod, organische Halogenverbindungen, vorzugsweise Jodide und Bromide, Komplexierungsreagentien, Schwermetallionen, Phosphate und Polyphosphate.

Geeignete Urease-Inhibitoren werden auch in J.Enz.lnhib. Bd. 16 (2001), Seiten 507 bis 516, J.Am.Chem.Soc. Bd.126 (2004), Seiten 3714 und 3715, J.Biol.Chem. Bd. 264 (1989), Seiten 15835 bis 15842, Current Medicinal Chemitry Bd. 9 (2002), Seiten 1323 bis 1348, Appl.Biochem.Microbio. Bd. 41 (2005), Seiten 23 bis 28, Biochemistry (Moscow) Bd. 69 (2004), Seiten 1344 bis 1352, Appl.Biochem.Microbio. Bd. 37 (2001), Seiten 168 bis 173, Food Chem. Bd. 85 (2004), Seiten 553 bis 558, Chem.Pharm.Bull. Bd. 51 (2003), Seiten 719 bis 723, Bioorg.Med.Chem. Bd. 12 (2004), Seiten 1963 bis 1968, und J.Enz.Inhib.Med.Chem. Bd. 19 (2004), Seiten 367 bis 371, beschrieben.

Bevorzugte Urease-Inhibitoren sind substituierte Thiophosphorsäuretriamide der Forrmel (I)

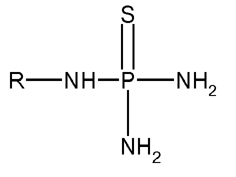

Beispiele für C₁- bis C₁₀-Alkylreste sind Methyl, Ethyl, n-Propyl, Isopropyl, n-Butyl, Isobutyl, sek.-Butyl, tert.-Butyl, n-Pentyl, Isopentyl, n-Hexyl, Isohexyl, n-Heptyl, Isoheptyl, n-Oktyl, Isooktyl, n-Nonyl, Isononyl, n-Dekyl und Isodekyl. Ganz besonders bevorzugte Alkylreste sind n-Propyl und n-Butyl.

Die substituierten Thiophosphorsäuretriamide der Formel (1) werden beispielsweise durch Umsetzung von Thiophosphoryltrichlorid mit Alkylamin und Ammoniak erhalten.

Der Herstellung der substituierten Thiophosphorsäuretriamide wird beispielsweise in US-5,770,771 beschrieben. Höhere Reinheiten werden erzielt, wenn die Produkte in einem geeigneten Lösungsmittel, beispielsweise Toluol, umkristallisiert werden.

Weiterhin bevorzugte Urease-Inhibitoren sind substituierte Phosphorsäuretriamide der Formel (II) 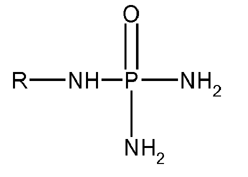 worin R die obengenannten Bedeutungen hat.

Die substituierten Phosphorsäuretriamide der Forrmel (II) entstehen beispielsweise durch Hydrolyse der Thiophosphorsäuretriamide der Forrmel (I).

Ein weiterhin bevorzugter Urease-Inhibitor ist auch Phenylphosphorodiamidat (CAS-Nr. 7450-69-3).

Die erfindungsgemäße Zusammensetzung enthält üblicherweise 0,0001 bis 0,1 Gew.-%, vorzugsweise 0,005 bis 0,08 Gew.-%, besonders bevorzugt 0,01 bis 0,06 Gew.-%, ganz besonders bevorzugt 0,015 bis 0,045 Gew.-%, des mindestens einen Urease-Inhibitors.

Die erfindungsgemäßen Zusammensetzungen haben eine hervorragende geruchsverhindernde Wirkung und eine hohe Absorptionskapazität.

Weitere Gegenstände der vorliegenden Erfindung sind Verfahren zur Herstellung der erfindungsgemäßen Zusammensetzungen, wobei man mindestens einen der folgenden Schritte durchführt:
i) mindestens ein Urease-Inhibitorwird mit mindestens einem wasserabsorbierenden Polymer zusammen gemischt,
ii) mindestens ein Urease-Inhibitor wird mit mindestens einem wasserabsorbierenden Polymer zusammen gemahlen,
iii) mindestens ein Urease-Inhibitor wird auf mindestens ein wasserabsorbierendes Polymer aufgesprüht,
iv) das mindestens eine wasserabsorbierende Polymer wird durch Lösungspolymerisation einer Monomerlösung hergestellt und mindestens ein Urease-Inhibitor wird in der Monomerlösung gelöst oder suspendiert.

Die Art des Mischens unterliegt keiner Beschränkung und kann bereits bei der Herstellung des wasserabsorbierenden Polymeren, beispielsweise beim Kühlen nach der Nachvernetzung oder dem anschließenden Sieben, oder in einem speziellen Mischer erfolgen. Geeignete Mischer wurden bereits oben bei der Nachvernetzung des wasserabsorbierenden Polymeren beschrieben.

Die Art des Mahlens unterliegt ebenfalls keiner Beschränkung. Geeignete Apparate wurden bereits oben bei der Zerkleinerung des wasserabsorbierenden Polymeren beschrieben.

Die Art des Aufsprühens unterliegt keiner Beschränkung. Der Urease-Inhibitor kann als Lösung oder als Schmelze aufgesprüht werden, beispielsweise während der Nachvernetzung des wasserabsorbierenden Polymeren in den dort genannten Mischern.

Vorteilhaft wird der mindestens eine Urease-Inhibitor in einem geeigneten Lösungsmittel gelöst aufgesprüht. Geeignete Lösungsmittel sind Wasser, Wasser/Aceton-Gemische, Wasser/Propylenglykol-Gemische sowie die bei der Nachvernetzung genannten Lösungsmittel und Lösungsmittelgemische. Die Konzentration des Urease-Inhibitor in der Lösung beträgt üblicherweise 0,5 bis 30 Gew.-%, vorzugsweise 1 bis 20 Gew.-%, besonders bevorzugt 2 bis 10 Gew.-%.

In einer weiteren Ausführungsform wird eine erfindungsgemäße Zusammensetzung hergestellt, die einen höheren Anteil des mindestens einen Urease-Inhibitors aufweist, üblicherweise 1 bis 50 Gew.-%, vorzugsweise 5 bis 40 Gew.-%, besonders bevorzugt 10 bis 30 Gew.-%. Die so erhaltene hochkonzentrierte Zusammensetzung kann dann mit weiterem wasserabsorbierenden Polymer auf den gewünschten Endgehalt verdünnt werden.

Weitere Gegenstände der vorliegenden Erfindung sind Hygieneartikel, enthaltend mindestens eine erfindungsgemäße Zusammensetzung, insbesondere Windeln oder Einlagen für schwere und/oder leichte Inkontinenz sowie Damenbinden, Babywindeln und Katzenstreu, und Verfahren zur Herstellung von Hygieneartikeln, wobei mindestens eine erfindungsgemäße Zusammensetzung verwendet wird.

Die erfindungsgemäßen wasserabsorbierenden Zusammensetzungen sind in der Lage unangenehme Gerüche, die in Hygieneartikel entstehen können, zuverlässig zu verhindern. Die erfindungsgemäßen Zusammensetzungen sind lagerstabil, so dass die geruchsbindende Wirkung auch noch nach längerer Lagerung, beispielsweise 6 Monate, vorhanden ist. Weiterhin weisen die erfindungsgemäßen Zusammensetzungen nach längerer Lagerung keine sichtbaren Verfärbungen auf.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

### Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

### Geruchsinhibierende Wirkung

Zur Bestimmung der geruchsverhindernden Wirkung wurde je 2 g der oben hergestellten Zusammensetzungen in einem 100 ml Erlenmeyerkolben vorgelegt und mit einer frisch bereiteten Lösung aus 30 mg Urease (aus Schwertbohnen; lyophilisiert 5 U/mg zur Harnstofbestimmung im Serum; Merck KGaA, DE) und 50 ml 0,9%iger Kochsalzlösung, wobei die Kochsalzlösung 8,56 g/l Harnstoff enthielt, versetzt und mit einem Stopfen mit innenliegenden Diffusionsröhrchen (Dräger® Röhrchen; Ammoniak 20 / a-D, 20 bis 1500 ppm*h) verschlossen. Nach 6 Stunden wurde der Messwert abgelesen. Die Messung wurde bei 23°C durchgeführt.

### Beispiele

### Beispiel 1

### Herstellung des wasserabsorbierenden Polymeren (Neutralisationsgrad 55 mol-%)

3708 g einer 37,3gew.-%igen wässrigen Natriumacrylatlösung wurden mit 867 g Acrylsäure und 1389 g Wasser gemischt und mit Stickstoff inertisiert. Diese Mischung wurde in einen mit Stickstoff inertisierten Werner & Pfleiderer LUK 8,0 K2 Kneter (2 Sigma-Wollen) eingefüllt und nacheinander mit 6,2 g Polyethylenglykoldiacrylat-400 (Diacrylat eines Polyethylenglykols mit einem mittleren Molgewicht von 400 g/mol), 15,4 g einer 0,5gew.-%igen wässrigen Ascorbinsäurelösung, 12,8 g einer 15gew.-%igen wässrigen Natriumpersulfatlösung und 1,5 g einer 2,5gew.-%igen wässrigen Wasserstoffperoxidlösung versetzt. Der Kneter wurde bei Maximaldrehzahl (98 upm der schnelleren Welle, ca. 49 upm auf der langsameren Welle, Verhältniss ca. 2:1) gerührt. Sofort nach der Zugabe von Wasserstoffperoxid wurde der Knetermantel mit 80°C warmen Wärmeträger beheizt. Bei einer Inntentemperatur von 60°C wurden 152 g SAP-Feinstaub mit einem Neutralisationsgrad von 65 mol.-% und einer Partikelgröße von weniger als 100 µm zugesetzt. Nach Erreichen der Maximaltemperatur wurde die Mantelheizung abgeschaltet und im Kneter weitere 15 Minuten nachreagieren lassen. Das Gel wurde auf 65°C abgekühlt und ausgefüllt. Die Trocknung des Gels erfolgte bei 155°C für 90 Minuten mit einer Beladung von 700 g pro Blech im Umlufttrockenschrank. Nach dreimaligem Mahlen in einem Walzenstuhl (Gebr. Baumeister LRC 125/70, Spaltbreiten 1000 µm, 600 µm, 400 µm) wurde das Polymer auf einen Siebschnitt zwischen 850 und 100 µm abgesiebt.

Die Zentrifugenretentionskapazität (CRC) der wasserabsorbierenden Polymere betrug 35,7 g/g.

1200 g dieses Polymers wurden in einen Gebr. Lödige Labormischer (Typ M5R) überführt. Bei Raumtemperatur wurde eine Mischung aus 12 g 1,2-Propandiol, 1,2 g Diethylenglykoldiglycidylether und 24 g Wasser über eine erste Düse und 12 g einer Aluminiumsulfatlösung (26,8 Gew.-% Al₂(SO₄)₃ in Wasser) über eine zweite Düse aufgesprüht. Anschließend wurde der Mischer schnell auf 150°C erwärmt und 30 Minuten bei dieser Temperatur gehalten. Nach dem Abkühlen wurde das Polymer auf einen Siebschnitt zwischen 850 und 100 µm abgesiebt.

Die Zentrifugenretentionskapazität (CRC) der oberflächennachvernetzten wasserabsorbierenden Polymere betrug 28,5 g/g.

### Beispiel 2

### Herstellung des wasserabsorbierenden Polymeren (Neutralisationsgrad 60 mol-%)

3993 g einer 37,3gew.-%igen wässrigen Natriumacrylatlösung wurden mit 761 g Acrylsäure und 1211 g Wasser gemischt und mit Stickstoff inertisiert. Diese Mischung wurde in einen mit Stickstoff inertisierten Werner & Pfleiderer LUK 8,0 K2 Kneter (2 Sigma-Wollen) eingefüllt und nacheinander mit 6,3 g Polyethylenglykoldiacrylat-400 (Diacrylat eines Polyethylenglykols mit einem mittleren Molgewicht von 400 g/mol), 15,2 g einer 0,5gew.-%igen wässrigen Ascorbinsäurelösung, 12,7 g einer 15gew.-%igen wässrigen Natriumpersulfatlösung und 1,5 g einer 2,5gew.-%igen wässrigen Wasserstoffperoxidlösung versetzt. Der Kneter wurde bei Maximaldrehzahl (98 upm der schnelleren Welle, ca. 49 upm auf der langsameren Welle, Verhältniss ca. 2:1) gerührt. Sofort nach der Zugabe von Wasserstoffperoxid wurde der Knetermantel mit 80°C warmen Wärmeträger beheizt. Bei einer Inntentemperatur von 60°C wurden 152 g SAP-Feinstaub mit einem Neutralisationsgrad von 60 mol.-% und einer Partikelgröße von weniger als 100 µm zugesetzt. Nach Erreichen der Maximaltemperatur wurde die Mantelheizung abgeschaltet und im Kneter weitere 15 Minuten nachreagieren lassen. Das Gel wurde auf 65°C abgekühlt und ausgefüllt. Die Trocknung des Gels erfolgte bei 155°C für 90 Minuten mit einer Beladung von 700 g pro Blech im Umlufttrockenschrank. Nach dreimaligem Mahlen in einem Walzenstuhl (Gebr. Baumeister LRC 125/70, Spaltbreiten 1000 µm, 600 µm, 400 µm) wurde das Polymer auf einen Siebschnitt zwischen 850 und 100 µm abgesiebt.

Die Zentrifugenretentionskapazität (CRC) der wasserabsorbierenden Polymere betrug 35,7 g/g.

1200 g dieses Polymers wurden in einen Gebr. Lödige Labormischer (Typ M5R) überführt. Bei Raumtemperatur wurde eine Mischung aus 12 g 1,2-Propandiol, 1,2 g Diethylenglykoldiglycidylether und 24 g Wasser über eine erste Düse und 12 g einer Aluminiumsulfatlösung (26,8 Gew.-% Al₂(SO₄)₃ in Wasser) über eine zweite Düse aufgesprüht. Anschließend wurde der Mischer schnell auf 160°C erwärmt und 40 Minuten bei dieser Temperatur gehalten. Nach dem Abkühlen wurde das Polymer auf einen Siebschnitt zwischen 850 und 100 µm abgesiebt.

Die Zentrifugenretentionskapazität (CRC) der oberflächennachvernetzten wasserabsorbierenden Polymere betrug 29,2 g/g.

### Beispiel 3

### Herstellung des wasserabsorbierenden Polymeren (Neutralisationsgrad 72 mol-%)

4809 g einer 37,3gew.-%igen wässrigen Natriumacrylatlösung wurden mit 534 g Acrylsäure und 573 g Wasser gemischt und mit Stickstoff inertisiert. Diese Mischung wurde in einen mit Stickstoff inertisierten Werner & Pfleiderer LUK 8,0 K2 Kneter (2 Sigma-Wollen) eingefüllt und nacheinander mit 4,8 g Polyethylenglykoldiacrylat-400 (Diacrylat eines Polyethylenglykols mit einem mittleren Molgewicht von 400 g/mol), 4,8 g 15-fach ethoxiliertem Trimethylolpropantriacrylat, 4,4 g einer 1,0gew.-%igen wässrigen Ascorbinsäurelösung, 18,1 g einer 15gew.-%igen wässrigen Natriumpersulfatlösung und 3,9 g einer 3gew.-%igen wässrigen Wasserstoffperoxidlösung versetzt. Der Kneter wurde bei Maximaldrehzahl (98 upm der schnelleren Welle, ca. 49 upm auf der langsameren Welle, Verhältniss ca. 2:1) gerührt. Sofort nach der Zugabe von Wasserstoffperoxid wurde der Knetermantel mit 80°C warmen Wärmeträger beheizt. Nach Erreichen der Maximaltemperatur wurde die Mantelheizung abgeschaltet und im Kneter weitere 15 Minuten nachreagieren lassen. Das Gel wurde auf 65°C abgekühlt und ausgefüllt. Die Trocknung des Gels erfolgte bei 175°C für 75 Minuten mit einer Beladung von 700 g pro Blech im Umlufttrockenschrank. Nach dreimaligem Mahlen in einem Walzenstuhl (Gebr. Baumeister LRC 125/70, Spaltbreiten 1000 µm, 600 µm, 400 µm) wurde das Polymer auf einen Siebschnitt zwischen 850 und 100 µm abgesiebt.

Die Zentrifugenretentionskapazität (CRC) der wasserabsorbierenden Polymere betrug 36,1 g/g.

1200 g dieses Polymers wurden in einen Gebr. Lödige Labormischer (Typ M5R) überführt. Bei Raumtemperatur wurde eine Mischung aus 12 g 1,2-Propandiol, 1,3 g Diethylenglykoldiglycidylether und 28 g Wasser über eine erste Düse und 12 g einer Aluminiumsulfatlösung (26,8 Gew.-% Al₂(SO₄)₃ in Wasser) über eine zweite Düse aufgesprüht. Anschließend wurde der Mischer schnell auf 168 °C erwärmt und 40 Minuten bei dieser Temperatur gehalten. Nach dem Abkühlen wurde das Polymer auf einen Siebschnitt zwischen 850 und 100 µm abgesiebt.

Die Zentrifugenretentionskapazität (CRC) der oberflächennachvernetzten wasserabsorbierenden Polymere betrug 30,0 g/g.

### Beispiel 4

Je 100 g wasserabsorbierendes Polymer aus Beispiel 1 wurden in einem Taumelmischer mit unterschiedlichen Mengen an Natriumfluorid je 20 Minuten gemischt. Anschließend wurde die Zentrifugenretentionskapazität (CRC) gemessen.

Die folgende Tabelle zeigt die Messergebnisse:

**Tab. 1: Natriumfluorid als Urease-Inhibitor**

| Konzentration in der Zusammensetzung | CRC |
|---|---|
| ohne | 28,5 g/g |
| 0,0500 Gew.-% | 29,1 g/g |
| 0,0900 Gew.-% | 28,5 g/g |
| 0,2000 Gew.-% | 24,1 g/g |

### Beispiel 5

Es wurde verfahren wie unter Beispiel 4. Statt Natriumfluorid wurde Hydrochinon verwendet.

**Tab. 2: Hydrochinon als Urease-Inhibitor**

| Konzentration in der Zusammensetzung | CRC |
|---|---|
| ohne | 28,5 g/g |
| 0,0050 Gew.-% | 28,5 g/g |
| 0,0500 Gew.-% | 28,7 g/g |
| 0,2000 Gew.-% | 25,3 g/g |
| 1,0000 Gew.-% | 23,4 g/g |

### Beispiel 6

Es wurde verfahren wie unter Beispiel 4. Statt Natriumfluorid wurde eine 80 gew.-%ige Mischung aus N-(n-Butyl)-thiophosphorsäuretriamid und N-(n-Propyl)-thiophosphorsäuretriamid (Gewichtsverhältnis 3 : 1) verwendet.

**Tab. 3: N-(n-Butyl)-thiophosphorsäuretriamid/N-(n-Propyl)-thiophosphorsäuretriamid als Urease-Inhibitor**

| Konzentration in der Zusammensetzung | CRC |
|---|---|
| ohne | 28,5 g/g |
| 0,0500 Gew.-% | 29,0 g/g |
| 0,0900 Gew.-% | 28,8 g/g |
| 0,2000 Gew.-% | 25,9 g/g |

### Beispiel 7

Je 100 g wasserabsorbierendes Polymer aus den Beispielen 1 bis 3 wurden in einem Taumelmischer mit unterschiedlichen Mengen an Hydrochinon je 20 Minuten gemischt. Anschließend wurde die geruchsinhibierende Wirkung gemessen.

Die folgende Tabelle zeigt die Messergebnisse:

**Tab. 4: Hydrochinon als Urease-Inhibitor (NH₃ nach 6 Stunden)**

| Konzentration in der Zusammensetzung | Neutralisationsgrad | | |
|---|---|---|---|
| | 55 mol-% | 60 mol-% | 72 mol-% |
| ohne | 120 ppm | 208 ppm | 850 ppm |
| 0,0025 Gew.-% | 91 ppm | 182 ppm | 800 ppm |
| 0,0050 Gew.-% | 58 ppm | 78 ppm | 381 ppm |
| 0,0100 Gew.-% | 29 ppm | 45 ppm | 214 ppm |
| 0,0200 Gew.-% | 0 ppm | 10 ppm | 63 ppm |
| 0,0500 Gew.-% | 0 ppm | 0 ppm | 15 ppm |
| 0,2000 Gew.-% | 0 ppm | 0 ppm | 0 ppm |
| 1,0000 Gew.-% | 0 ppm | 0 ppm | 0 ppm |

### Beispiel 8

Es wurde verfahren wie unter Beispiel 7. Statt Hydrochinon wurde eine 80 gew.-%ige Mischung aus N-(n-Butyl)-thiophosphorsäuretriamid und N-(n-Propyl)-thiophosphorsäuretriamid (Gewichtsverhältnis 3 : 1) verwendet.

**Tab. 5: N-(n-Butyl)-thiophosphorsäuretriamid/N-(n-Propyl)-thiophosphorsäuretriamid als Urease-Inhibitor (NH₃ nach 6 Stunden)**

| Konzentration in der Zusammensetzung | Neutralisationsgrad | |
|---|---|---|
| | 55 mol-% | 72 mol-% |
| ohne | 120 ppm | 850 ppm |
| 0,0100 Gew.-% | 25 ppm | 49 ppm |
| 0,0300 Gew.-% | 7 ppm | 31 ppm |
| 0,0500 Gew.-% | 0 ppm | 6 ppm |
| 0,0900 Gew.-% | 0 ppm | 0 ppm |
| 0,2000 Gew.-% | 0 ppm | 0 ppm |

## Patentansprüche

1. Zusammensetzung, enthaltend mindestens ein wasserabsorbierendes Polymer und mindestens einen Urease-Inhibitor, wobei das Polymer ein Säure-Gruppen tragendes Polymer ist, dessen Säure-Gruppen zu 50 bis 65 mol-% neutralisiert sind, und der Gehalt an Urease-Inhibitor von 0,0001 bis 0,1 Gew.-%, bezogen auf die Zusammensetzung, beträgt.

2. Zusammensetzung gemäß Anspruch 1, **dadurch gekennzeichnet, dass** die Säure-Gruppen des wasserabsorbierenden Polymeren zu 54 bis 61 mol-% neutralisiert sind und/oder der Gehalt an Urease-Inhibitor von 0,015 bis 0,045 Gew.-%, bezogen auf die Zusammensetzung, beträgt.

3. Zusammensetzung gemäß Anspruch 1 oder 2, **dadurch gekennzeichnet, dass** die Zusammensetzung mindestens 90 Gew.-% wasserabsorbierendes Polymer enthält.

4. Zusammensetzung gemäß einem der Ansprüche 1 bis 3, **dadurch gekennzeichnet, dass** das wasserabsorbierende Polymer ein Polymer auf Basis einer vernetzten Acrylsäure ist.

5. Zusammensetzung gemäß einem der Ansprüche 1 bis 4, **dadurch gekennzeichnet, dass** das wasserabsorbierende Polymer im Form oberflächennachvernetzter Partikel vorliegt.

6. Verfahren zur Herstellung der in den Ansprüchen 1 bis 5 definierten Zusammensetzungen, **dadurch gekennzeichnet, dass** man mindestens einen der folgenden Schritte durchführt:
i) mindestens ein Urease-Inhibitor wird mit mindestens einem wasserabsorbierenden Polymer zusammen gemischt,
ii) mindestens ein Urease-Inhibitor wird mit mindestens einem wasserabsorbierenden Polymer zusammen gemahlen,
iii) mindestens ein Urease-Inhibitor wird auf mindestens ein wasserabsorbierendes Polymer aufgesprüht,
iv) das mindestens eine wasserabsorbierende Polymer wird durch Lösungspolymerisation einer Monomerlösung hergestellt und mindestens ein Urease-Inhibitor wird in der Monomerlösung gelöst oder suspendiert.

7. Verfahren zur Herstellung der in den Ansprüchen 1 bis 5 definierten Zusammensetzungen, **dadurch gekennzeichnet, dass** man mindestens einen der folgenden Schritte durchführt:
i) mindestens ein Urease-Inhibitor wird mit mindestens einem wasserabsorbierenden Polymer zusammen gemischt,
ii) mindestens ein Urease-Inhibitor wird mit mindestens einem wasserabsorbierenden Polymer zusammen gemahlen,
iii) mindestens ein Urease-Inhibitor wird auf mindestens ein wasserabsorbierendes Polymer aufgesprüht,
iv) das mindestens eine wasserabsorbierende Polymer wird durch Lösungspolymerisation einer Monomerlösung hergestellt und mindestens ein Urease-Inhibitor wird in der Monomerlösung gelöst oder suspendiert,
und die in einer ersten Stufe (I) gemäß i), ii), iii) und/oder iv) erhaltene Zusammensetzung in einer zweiten Stufe (II) mit mindestens einem wasserabsorbierenden Polymer zusammen gemischt wird.

8. Verfahren gemäß Anspruch 7, **dadurch gekennzeichnet, dass** die Zusammensetzung nach Stufe (I) und vor Stufe (II) von 1 bis 50 Gew.-% des mindestens einen Urease-Inhibitors, bezogen auf die Zusammensetzung, enthält.

9. Hygieneartikel, enthaltend mindestens eine Zusammensetzung gemäß einem der Ansprüche 1 bis 5.

10. Hygieneartikel gemäß Anspruch 9, **dadurch gekennzeichnet, dass** der Hygieneartikel eine Windel oder Einlage für schwere oder leichte Inkontinenz, ein Damenbinden, eine Babywindel oder Katzenstreu ist.
